# EUROPEAN PATENT SPECIFICATION

(11) **EP 4 181 976 B1**
(45) Date of publication and mention of the grant of the patent: **20.08.2025**
(21) Application number: 21765699.0
(22) Date of filing: 15.07.2021
(51) Int. Cl.: A61L 9/12, A62B 23/00, A41D 13/00, A41D 27/08, A62B 23/02

(54) **ACCESSORY DEVICE FOR FACE COVERINGS**
ZUBEHÖRVORRICHTUNG FÜR GESICHTSABDECKUNGEN
DISPOSITIF ACCESSOIRE POUR COUVRE-VISAGES

(30) Priority: 20.07.2020 IT 202000004315 U
(43) Date of publication of application: 24.05.2023
(73) Proprietor: I.T.P. S.r.l. Innovation & Technology Provider, 80121 Napoli (IT)
(72) Inventor: ZINO, Maria Rosa Viola, 80126 Napoli (IT); NIGRO, Alessandro, 80126 Napoli (IT)
(74) Representative: Soldatini, Andrea
(86) International application number: PCT/IB2021/056381
(87) International publication number: WO 2022/018583

(56) References cited:
- WO-A1-2005/044381
- WO-A2-2011/081936
- WO-A2-2015/006679
- JP-U- H 071 806
- US-A1- 2006 032 026
- US-A1- 2009 145 434
- US-A1- 2012 090 616

## Description

### Field of the Invention

The present invention relates to an accessory device for face coverings, surgical masks and similar medical and personal protective equipment, such as filtering facial devices.

### State of the Art

In view of the recent health emergency caused by the spread of the SARS-COV2 virus, the use of surgical masks or of filtering facepieces has been made compulsory by law and, even where it has not been made compulsory, a precautionary use of such devices is considered advisable in closed and/or crowded environments, encouraged by the medical and scientific community, but also requested by the citizens themselves. Similar situations have already occurred in the past, for example during the H1N1 virus emergency in 2009, and, looking to the future, the use of such devices appears to be an indispensable instrument for civil coexistence in a globalised world.

On the one hand, these devices have proved to be effective protection means, on the other hand with the practically daily use of these devices, it soon became apparent that there were certain shortcomings or that certain aspects needed to be improved.

One of these is the need to be able to recognise one's mask when one has to take it off temporarily and then put it on again. There are situations in which the mask has to be removed and placed, for example, on the desk in the office or on a countertop in a public place in order to carry out certain operations; when the mask is to be put on again, it cannot be recognised for sure, especially if other masks of the same type have been placed near it. Situations like this are very frequent and clearly imply a high risk of spreading the virus.

The difficult recognizability of the person who wears the face covering is also another aspect to be improved. In fact, if worn in normal conditions, the face covering leaves uncovered only the upper part of the face. This inconvenience is even more evident when the person who wears the face covering also wears accessories, such as glasses and hats.

Another undesirable aspect connected with the use of these protection devices is represented by the unpleasant odours that can be concentrated inside the face covering, due in particular to the user's bad breath. The need to wear the face covering so as to cover the user's nose and mouth obliges the user to inhale and exhale air in the narrow space created by the face covering in front of the mouth, so bad breath can be particularly unpleasant, and in some cases unbearable, when wearing the face covering.

Another aspect to be taken into account is the microbiological profile that is established inside the face covering. This microbiological profile is the result of the sum of the microbiological load, naturally residing on the internal surface of the face covering due to the production process, and of the microbiological load released by the user during breathing and during the interaction between the face covering and the cheeks, nose and mouth apparatus. During use this microbiological load tends to increase due to the favourable microclimatic conditions (controlled temperature of approximately 36-37°C and humidity close to RH 100%), up to values that can even lead to the onset of undesirable pathological events. This consideration is even more critical when considering the prolonged reuses of the face covering.

The need to overcome these drawbacks is therefore very much felt.

A diffuser and identification device for face coverings having the features in the preamble of independent claim 1 attached is disclosed by document US2012/090616.

### Summary of the invention

Subject of this invention is precisely to provide a device that functions both as a diffuser, in particular of fragrances, scents and essential oils, and as a recognition signal for surgical masks and filtering facepieces.

A further subject of the present invention is to allow the identification of a user, for instance as member of an organization, a company, an operative department or of a specific group.

A further subject of the present invention is to provide the aforesaid device that is also reusable once the face covering or the facial device is exhausted and to be replaced with a new protection device.

A further subject of this invention is to provide a face covering, surgical mask or filtering facepiece provided with the aforesaid device, which is comfortable, as well as effective in eliminating unpleasant odours and helping in finding its own face covering, and is neither bulky nor presents discomfort during use.

A further subject of this invention is the possibility, through the use of a diffuser element containing natural and/or synthetic essential oils with antibacterial and antiviral active ingredients, to reduce the bacterial and viral load, which, as a result of prolonged use and the particular microclimatic conditions created inside the face coverings, tends to increase and can therefore trigger pathological events generated by the increasing microbial and/or viral load.

A further subject of the present invention is to provide a kit comprising at least one face covering or surgical mask or filtering facepiece and at least one aforesaid device with function of diffuser and identification signal.

These subjects are achieved with the present accessory device, with dual function of diffuser and of identification of the user, for face coverings, surgical masks and for filtering facepieces, as claimed in the annexed claims and described in the following, also with reference to the figures.

### Brief Description of the Figures

The invention will now be illustrated more in detail with the description that follows of an embodiment thereof, provided by way of non-limiting example with reference to the accompanying drawings in which:
- Figures 1 to 5 show several non-limiting examples of an identification element comprised in the present device, each with a front perspective view "a" and a rear perspective view "b";
- Figure 6 schematically shows an embodiment of the present device as a whole, with the diffuser and identification elements facing each other, on opposite sides of a face covering, in an approach phase (part a) and in place during use (part b);
- Figures 7a and 7b show, respectively, the inside of a face covering to which the diffuser element of the present device is affixed, and the outside of the same face covering with the decorative flower-shaped identification element, placed on the face covering;
- Figures 8-10 show different and alternative embodiments of a diffuser element for a device according to the present invention;
- Figure 11 shows an example of an identification element of the device of this invention, with a front perspective view "a" and a rear perspective view "b", comprising the proprietary logo of the Applicant engraved on the front as a decorative and identification element; in Figure 11 is also showed an exploded view "c" of this element, where magnets placed inside the identification element are visible;
- Figure 12 shows an example of diffuser element in the device of the invention, also comprising the proprietary logo of Figure 11 engraved on the front, as a decorative and identification element, and a rear metal surface intended to be coupled to an identification element equipped with magnets and placed on the opposite side with respect to a face covering, such as that showed in Figure 11.

### Detailed Description of the Invention

The accessory device, with dual function of diffuser and identifier, for face coverings, surgical masks and filtering facial devices according to the invention, comprises at least an identification element 1 comprising a decorative element 11 intended to be affixed to the external surface of a face covering or filtering facepiece, and at least a diffuser element 2 comprising a container intended to be affixed to the internal surface of the same face covering, mask or filtering facepiece, having at least part of an exposed surface thereof perforated 21, said elements 1 and 2 each being provided with magnetic means for their mutual coupling and uncoupling on opposite sides of a face covering, mask or a filtering facepiece.

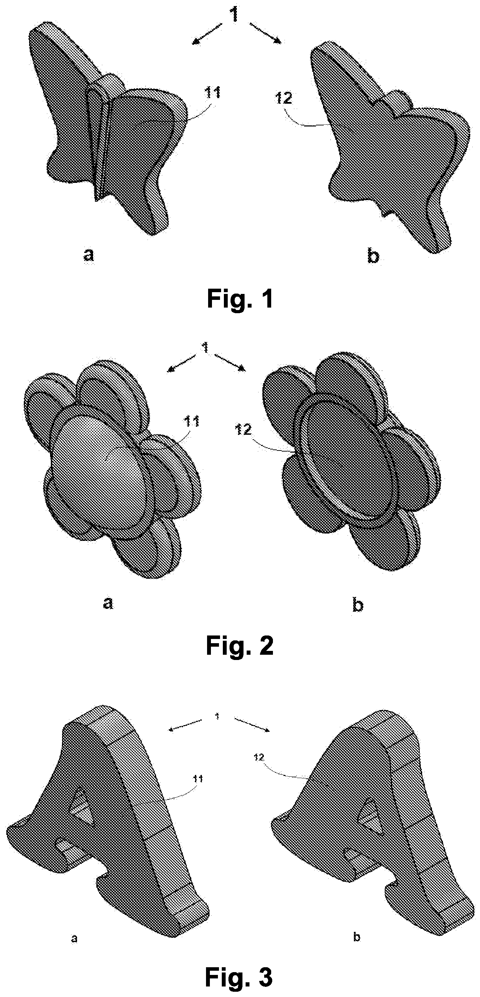

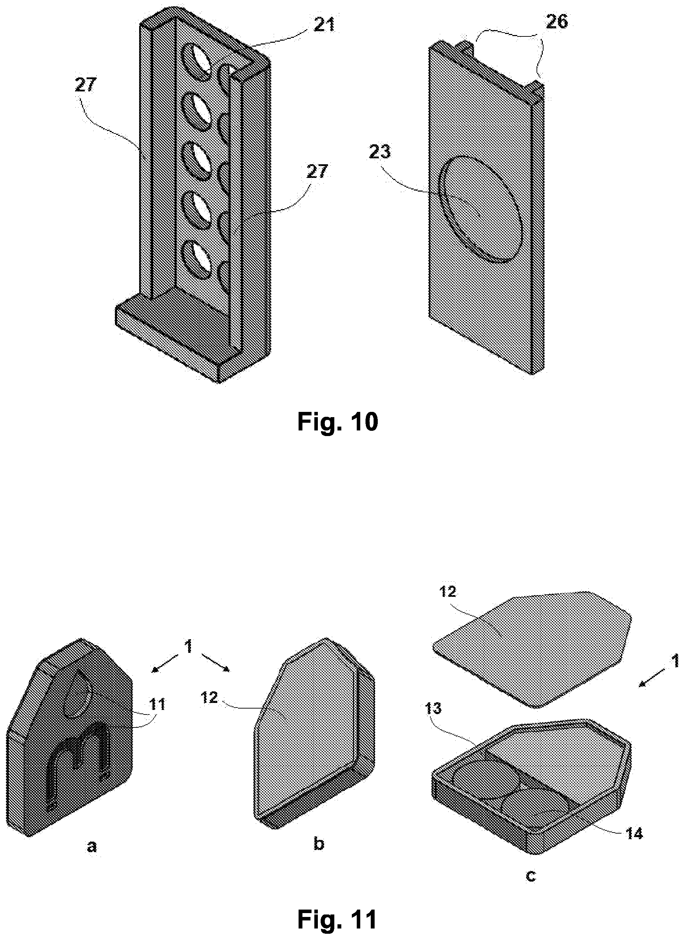

With reference to the aforesaid figures, the identification element is generically indicated by 1 and the diffuser element of the present device by 2. The identification element 1, intended for the positioning on the external face of the face covering, consists of a three-dimensional element 11, which may have various shapes and thus also a decorative function. Non-limiting examples of identification elements 1 comprised in the present device are shown in Figures 1 to 5 and in Figure 11; in each of these Figures 1 to 5 and 11, the identification element 1 is shown from the front (part a) to highlight the decorative element 11 and from the back (part b) to show the rear surface 12, opposite to the element 11, and intended for contact with the face covering and, through this, with the rear surface of the diffuser container further described below. The surface 12 may be a flat metal surface, like in Figures 1 and 3-5, or it may be a surface provided with a housing 13 for magnets 14, like in Figures 2 and 11.

For the avoidance of doubts, when herein reference is made to a rear or posterior part/surface of the identification element 1 and/or of the diffuser element 2 that part or surface is meant that is intended for being in contact with the face covering, while by front or anterior part/surface is meant the opposite part or surface, from the face covering towards the exterior, on both surfaces of the face covering.

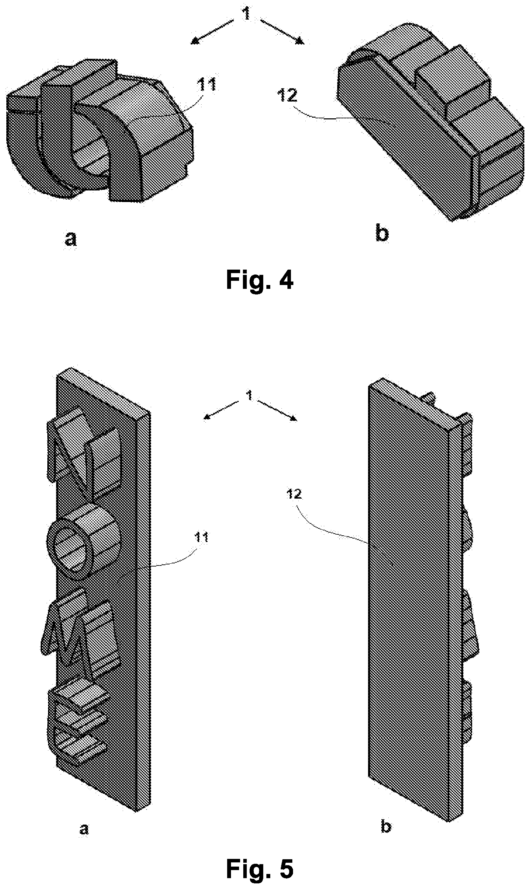

The decorative or identification figure represented in element 1 of the present device can be a plate with the name or with the initials of the name of the user of the face covering, or it can be a fantasy figure, a flower, an animal, a miniature photo holder support, a logo, for example the Applicant's logo like in Figure 4 attached herein. In addition to being a signal of recognisability of the face covering, the element 1 can also have a decorative function, making the appearance of the protection device more attractive. In an aspect of the present invention, the decorative or identification figures can be applied on an element of the device by any means, or they can be directly drawn or engraved in the element or embossed on it.

According to a particular embodiment of the present invention, the element 1 of the device comprises one or more decorative or identification figures 11 selected from the group consisting of graphic signs, initials, images, photographs or graphic codes, smart electronic elements of active and/or passive type. One or more elements of this type allow the identification or recognition of the user by third parties, but also how to distinguish its own face covering by the user her/himself, who temporarily has taken off the face covering and would like to wear it again.

The device of this invention comprises, in addition to an identification element 1, a diffuser element 2, suitable for diffusing in particular fragrances and scents inside the face covering, but also essential oils, or natural active ingredients in the form of gel, cream or ointment, optionally absorbed on porous supports, for example on zeolites or on paper. Preferred are products of natural origin with antiviral, antifungal or antibacterial activity, which can help reduce the external and internal microbial load and any viral load possibly present. Such a diffuser element 2 consists of a container having an internal housing for the scents and/or the essential oils to be diffused and at least one perforated exposed surface 21, through which it diffuses scents and vapours. In one embodiment, an opening 22 allows the insertion of the scents to be diffused inside the container.

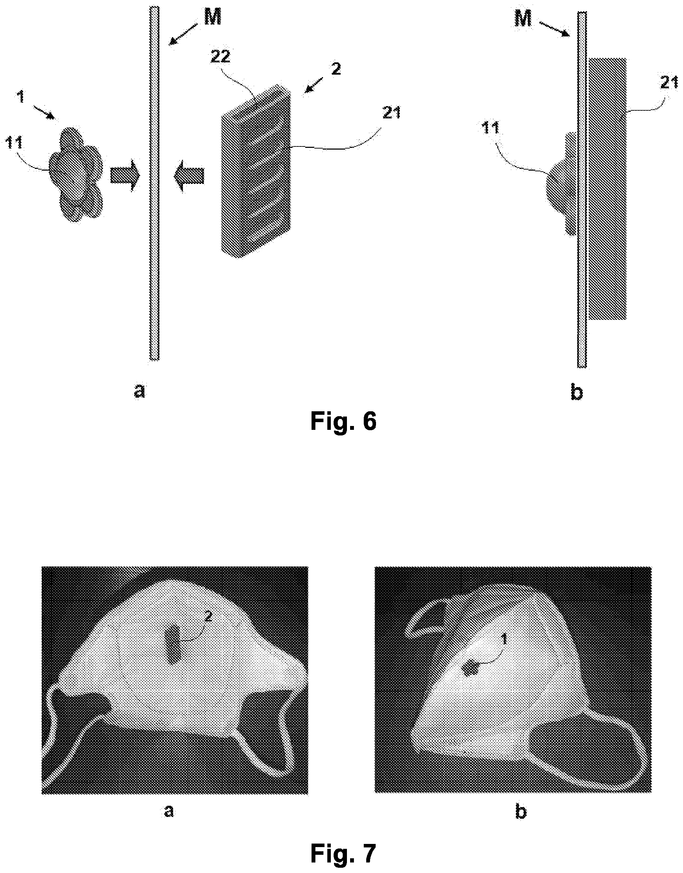

The magnetic means in the present accessory device for face coverings may be two magnets one on each element 1 and 2, or a magnet on one of the two elements 1 or 2 and a metal surface on the other element, as it is for the identification element in Figures 1 and 3-5. In an aspect, the magnets for use in the present device are neodymium magnets, e.g. tablet-shaped magnets. Figure 6 in particular shows how the two elements 1 and 2 of the present device are coupled, facing to each other, enclosing a face covering between them. In a preferred embodiment of the present device, these magnetic means are housed on the elements 1 and 2 on the opposite side with respect to the decorative figure 11 and to the perforated exposed surface 21 respectively, so as to maximise the diffusion of the scented or otherwise active substances within the face covering and to increase the visibility of the decorative figure from the outside.

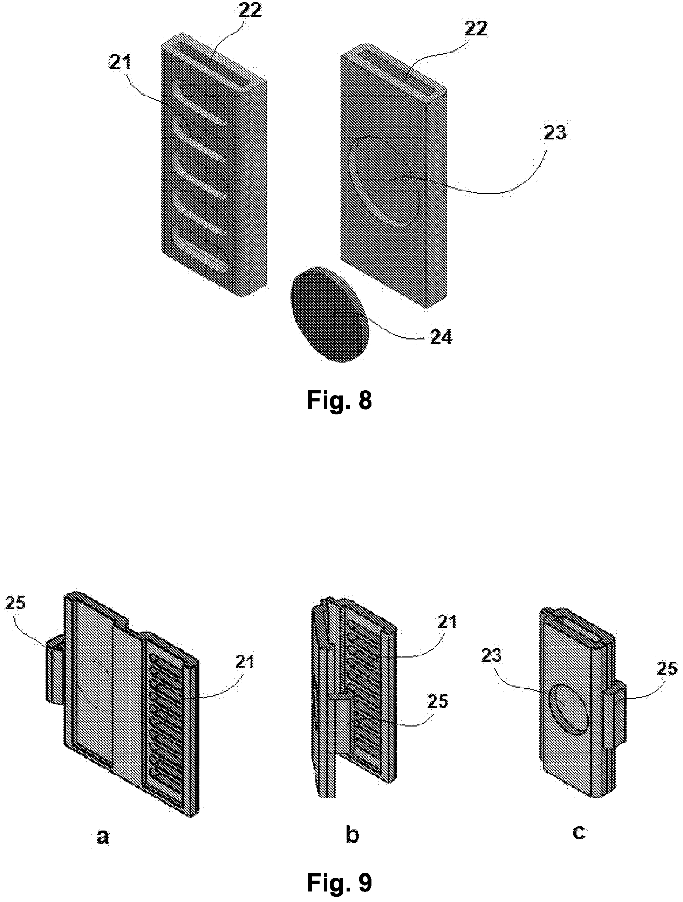

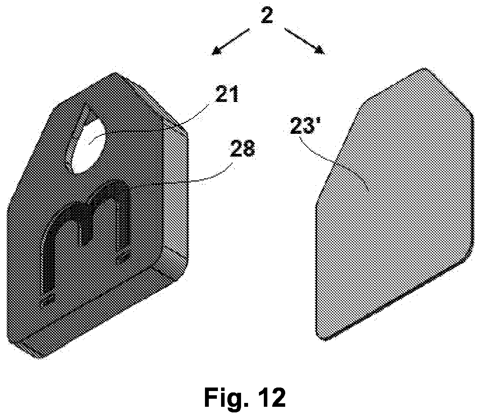

With reference to Figure 8, an embodiment of the element 2 is illustrated, consisting of a hollow body with a perforated surface 21, an opening 22 through which the scents to be diffused in the hollow body can be inserted, and a housing 23 for a magnet 24 formed on the surface intended for contact with the face covering and, through it, with the rear surface 12 of an identification element 1. In a particular embodiment of the invention, exemplified in Figure 12, the element 2 has a rear surface 23', that is in this case a metal surface, intended to be coupled with an element 1 equipped with magnets.

An alternative embodiment of the diffuser element 2 according to this invention may provide for an opening with hinge and door, as illustrated in Figure 9, where the element 2 is depicted in three different door opening and closing positions, a (fully open), b (semi-open), and c (closed). The door can in this case be advantageously fitted with a hook 25 for the snap closure of the door. This type of opening can facilitate the replacement of exhausted scent cartridges or the replacement thereof.

A further embodiment of the diffuser element 2 according to the present invention may instead provide for an interlocking opening, as illustrated in Figure 10, where the two portions forming the element 2 are separately represented, a first portion comprising the perforated surface 21 and a second rear portion comprising the housing 23 for the magnet and protruding profiles 26 adapted to interlock by sliding inside the edges 27 on the sides of the first portion.

In a particular embodiment of the present device, one example of which is illustrated in Figure 12, also the element 2 comprises a decorative or identification figure 28, that can be attached onto or directly engraved in the element 2. The decorative or identification figure 28 can also be selected among graphic signs, initials, images, photographs or graphic codes, smart electronic elements of active and/or passive type.

The device of the present invention has several advantages, in addition to optimally fulfilling its dual function of diffusing fragrances inside the face covering and allowing the recognition thereof by the user thanks to the external decorative element. Firstly, it is very easy to apply and use, and allows the positioning of the two elements anywhere on the body of the face covering according to the user's preference. This device is also comfortable, and the internal overall dimensions of the diffuser element are reduced to a minimum. The present device also fits any type of facial protection device, be it a surgical mask or a filtering facepiece, and is reusable even after the face covering is exhausted and needs to be replaced. The two elements of this device can be easily detached and repositioned for the face covering replacement or washing thereof, in the models that can be washed.

The device according to the invention may be provided in a kit further comprising a packaging pouch intended to further contain at least one face covering or filtering facepiece, sealed on the perimeter and preferably provided with an indentation or other means to facilitate the opening.

The invention is not limited to the embodiment described and illustrated above, but comprises any execution variants thereof within the scope of the attached claims.

## Claims

1. A diffuser and identification device for face coverings, surgical masks and for filtering facial devices, comprising at least an identification element (1) comprising a decorative or identification figure (11) intended to be affixed to the external surface of face covering, surgical mask or filtering facepiece, and at least a diffuser element (2) comprising a container intended to be affixed to the internal surface of the same mask or filtering facepiece, said container having an exposed surface at least part (21) of which is perforated, **characterized in that** said identification element (1) and said diffuser element (2) are each further provided with magnetic means for their mutual coupling and uncoupling on opposite sides of a face covering, surgical mask or of a filtering facepiece.

2. The device according to claim 1, wherein said magnetic means are two magnets one on each of said identification element (1) and said diffuser element (2), or a magnet on one of the identification element (1) or the diffuser element (2), and a ferromagnetic metal surface on the other element.

3. The device according to claim 1 or 2, wherein said magnetic means are housed on said identification element (1) and said diffuser element (2) on the opposite side with respect to said decorative figure (11) and to said perforated exposed surface (21), respectively.

4. The device according to any one of claims 1-3, wherein said diffuser element (2) is a hollow body with an opening (22) through which the substances to be diffused are introduced.

5. The device according to any one of claims 1-3, wherein said diffuser element (2) is a container that can be opened by interlocking or by means of a door with snap closure.

6. The device according to any one of the preceding claims, wherein said diffuser element (2) is adapted to diffuse through said perforated surface (21) scents, fragrances, essential oils, natural extracts in the form of gels, creams, ointments, optionally absorbed on porous supports such as paper or zeolites.

7. The device according to any one of the preceding claims, wherein said diffuser element (2) comprises a decorative or identification figure (28).

8. The device according to any one of the preceding claims, wherein said decorative or identification figure (11,28) is selected from among tags with name or initials of a user, fictional figures and graphic signs, miniature photo frames, logos, graphic codes, images, photographs and smart technology electronic elements of active and/or passive type.

9. A face covering, surgical mask or filtering facepiece comprising at least a device as defined in claims 1-8, positioned at the area of the mask intended to cover the mouth of the user with said identification figure facing outwards and said scent container facing inwards from the mask during the correct use thereof, said figure and said container being coupled together and held in place by means of respective magnets.

10. A kit comprising at least one face covering, surgical mask or filtering facepiece and at least one device as defined in claims 1-8.

## Patentansprüche

1. Diffusor- und Identifikationsvorrichtung für Gesichtsbedeckungen, chirurgische Masken und für Filtergesichtsvorrichtungen, umfassend mindestens ein Identifikationselement (1), umfassend eine dekorative oder eine Identifikationsfigur (11), die dafür vorgesehen ist, an der Außenoberfläche einer Gesichtsbedeckung, einer chirurgischen Maske oder einem Filtergesichtsstück angebracht zu werden, und mindestens ein Diffusorelement (2), umfassend einen Behälter, der dafür vorgesehen ist, an der Innenoberfläche derselben Maske oder desselben Filtergesichtsstücks angebracht zu werden, wobei der Behälter eine freiliegende Oberfläche aufweist, von der mindestens ein Teil (21) perforiert ist, **dadurch gekennzeichnet, dass** das Identifikationselement (1) und das Diffusorelement (2) jeweils weiter mit magnetischen Mitteln für ihre gegenseitige Kopplung und Entkopplung auf gegenüberliegenden Seiten einer Gesichtsbedeckung, einer chirurgischen Maske oder einem Filtergesichtsstück versehen ist.

2. Vorrichtung nach Anspruch 1, wobei die magnetischen Mittel zwei Magnete sind, jeweils einer auf dem Identifikationselement (1) und dem Diffusorelement (2), oder ein Magnet auf einem des Identifikationselements (1) oder des Diffusorelements (2) und eine ferromagnetische Metalloberfläche auf dem anderen Element.

3. Vorrichtung nach Anspruch 1 oder 2, wobei die magnetischen Mittel auf dem Identifikationselement (1) und dem Diffusorelement (2) auf der gegenüberliegenden Seite in Bezug auf die dekorative Figur (11) bzw. die perforierte freiliegende Oberfläche (21) untergebracht sind.

4. Vorrichtung nach einem der Ansprüche 1 bis 3, wobei das Diffusorelement (2) ein Hohlkörper mit einer Öffnung (22) ist, durch die die zu diffundierenden Substanzen eingeführt werden.

5. Vorrichtung nach einem der Ansprüche 1 bis 3, wobei das Diffusorelement (2) ein Behälter ist, der durch Verriegelung oder mittels einer Tür mit Schnappverschluss geöffnet werden kann.

6. Vorrichtung nach einem der vorstehenden Ansprüche, wobei das Diffusorelement (2) geeignet ist, um durch die perforierte Oberfläche (21) Düfte, Duftstoffe, ätherische Öle, natürliche Extrakte in Form von Gelen, Cremes, Salben, die optional auf porösen Trägern wie Papier oder Zeolithen absorbiert sind, zu diffundieren.

7. Vorrichtung nach einem der vorstehenden Ansprüche, wobei das Diffusorelement (2) eine dekorative oder eine Identifikationsfigur (28) umfasst.

8. Vorrichtung nach einem der vorstehenden Ansprüche, wobei die dekorative oder Identifikationsfigur (11, 28) aus Tags mit Namen oder Initialen eines Benutzers, fiktiven Figuren und grafischen Zeichen, Miniaturfotorahmen, Logos, grafischen Codes, Bildern, Fotografien und elektronischen Elementen mit intelligenter Technik aktiver und/oder passiver Art ausgewählt ist.

9. Gesichtsbedeckung, chirurgische Maske oder Filtergesichtsstück, umfassend mindestens eine Vorrichtung wie in den Ansprüchen 1 bis 8 definiert, die an dem Bereich der Maske positioniert ist, der dafür vorgesehen ist, den Mund des Benutzers mit der Identifikationsfigur abzudecken, die nach außen gewandt ist, und wobei der Duftbehälter von der Maske nach innen gewandt ist, während des korrekten Gebrauchs von dieser, wobei die Figur und der Behälter zusammen gekoppelt sind und mittels jeweiligen Magneten an Ort und Stelle gehalten werden.

10. Kit, umfassend mindestens eine Gesichtsbedeckung, eine chirurgische Maske oder ein Filtergesichtsstück und mindestens eine Vorrichtung wie in den Ansprüchen 1 bis 8 definiert.

## Revendications

1. Dispositif diffuseur et d'identification pour des couvre-visages, des masques chirurgicaux et des dispositifs faciaux filtrants, comprenant au moins un élément d'identification (1) comprenant une figure décorative ou d'identification (11) destinée à être apposée sur la surface externe du couvre-visage, du masque chirurgical ou du masque filtrant, et au moins un élément diffuseur (2) comprenant un récipient destiné à être apposé sur la surface interne du même masque ou du masque filtrant, ledit récipient ayant une surface exposée dont au moins une partie (21) est perforée, **caractérisé en ce que** ledit élément d'identification (1) et ledit élément diffuseur (2) sont en outre chacun pourvus de moyens magnétiques pour leur accouplement et désaccouplement mutuels sur des côtés opposés d'un couvre-visage, d'un masque chirurgical ou d'un masque filtrant.

2. Dispositif selon la revendication 1, dans lequel lesdits moyens magnétiques sont deux aimants, un sur chacun dudit élément d'identification (1) et dudit élément diffuseur (2), ou un aimant sur l'un de l'élément d'identification (1) ou de l'élément diffuseur (2), et une surface métallique ferromagnétique sur l'autre élément.

3. Dispositif selon la revendication 1 ou 2, dans lequel lesdits moyens magnétiques sont logés sur ledit élément d'identification (1) et ledit élément diffuseur (2) sur le côté opposé à ladite figure décorative (11) et à ladite surface exposée perforée (21), respectivement.

4. Dispositif selon l'une quelconque des revendications 1 à 3, dans lequel ledit élément diffuseur (2) est un corps creux doté d'une ouverture (22) par laquelle sont introduites les substances à diffuser.

5. Dispositif selon l'une quelconque des revendications 1 à 3, dans lequel ledit élément diffuseur (2) est un récipient qui peut être ouvert par verrouillage ou au moyen d'une porte à fermeture par encliquetage.

6. Dispositif selon l'une quelconque des revendications précédentes, dans lequel ledit élément diffuseur (2) est adapté à diffuser à travers ladite surface perforée (21) des senteurs, des parfums, des huiles essentielles, des extraits naturels sous forme de gels, de crèmes, d'onguents, éventuellement absorbés sur des supports poreux tels que du papier ou des zéolithes.

7. Dispositif selon l'une quelconque des revendications précédentes, dans lequel ledit élément diffuseur (2) comprend une figure décorative ou d'identification (28).

8. Dispositif selon l'une quelconque des revendications précédentes, dans lequel ladite figure décorative ou d'identification (11, 28) est choisie parmi des étiquettes portant le nom ou les initiales d'un utilisateur, des figures fictives et des signes graphiques, des cadres photo miniatures, des logos, des codes graphiques, des images, des photographies et des éléments électroniques de technologie intelligente de type actif et/ou passif.

9. Couvre-visage, masque chirurgical ou masque filtrant comprenant au moins un dispositif selon les revendications 1 à 8, positionné au niveau de la zone du masque destinée à couvrir la bouche de l'utilisateur avec ladite figure d'identification faisant face vers l'extérieur et ledit récipient à senteurs faisant face vers l'intérieur du masque pendant l'utilisation correcte associée, ladite figure et ledit récipient étant accouplés ensemble et maintenus en place au moyen d'aimants respectifs.

10. Kit comprenant au moins un couvre-visage, un masque chirurgical ou un masque filtrant et au moins un dispositif selon les revendications 1 à 8.
